# EUROPEAN PATENT SPECIFICATION

(11) **EP 4 117 559 B1**
(45) Date of publication and mention of the grant of the patent: **29.04.2026**
(21) Application number: 21716009.2
(22) Date of filing: 10.03.2021
(51) Int. Cl.: A61B 18/14, A61B 17/42

(54) **ROBOTIC COLPOTOMY SYSTEMS**
ROBOTISCHE KOLPOTOMIESYSTEME
SYSTÈMES ROBOTIQUES DE COLPOTOMIE

(30) Priority: 11.03.2020 US 202062987926 P
(43) Date of publication of application: 18.01.2023
(73) Proprietor: Covidien LP, Mansfield, MA 02048 (US)
(72) Inventor: PRIOR, Scott J., North Haven, Connecticut 06473 (US); BEGG, Nikolai D., Woburn, Massachusetts 01801 (US); RAJAGOPALAN MOHAN, Arvind, Woburn, Massachusetts 01801 (US); TRAINA, Zachary, Verona, New Jersey 07044 (US); SLISZ, Kevin R., North Haven, Connecticut 06473 (US)
(74) Representative: Halliwell, Bethan Frances
(86) International application number: PCT/US2021/021641
(87) International publication number: WO 2021/183607

(56) References cited:
- EP-A2- 3 400 894
- US-A1- 2018 325 575

## Description

### TECHNICAL FIELD

This disclosure relates to hysterectomy and, more particularly, to systems for performing a colpotomy procedure.

### BACKGROUND

Colpotomy, one of the final steps in a hysterectomy, requires making a circular incision in vaginal tissue to separate the uterus from the vagina with a cutting tool such as a electrosurgical instrument. This incision is typically performed with the aid of a uterine manipulator. Uterine manipulators are conventionally used during laparoscopic hysterectomy procedures to position the vagina and the cervix to facilitate separation and to enable removal of the uterus or other tissue specimens subsequent to performance of a colpotomy. EP3400894A2 discloses systems, devices, and methods for performing a colpotomy procedure.

### SUMMARY OF THE INVENTION

The invention is defined in claim 1. Any methods disclosed hereinafter do not form part of the scope of the invention, and are mentioned for illustrative purposes only.

### SUMMARY OF THE DISCLOSURE

In accordance with an aspect of this disclosure, an electrosurgical colpotomy system includes an energy source and a uterine manipulator. The uterine manipulator extends to a distal tip and supports a colpotomy cup. The colpotomy cup has an active electrode in electrical communication with the energy source. The active electrode is positioned to simultaneously treat a circumference of tissue in contact therewith.

In aspect, the colpotomy cup may include one or more return electrodes positioned thereon in electrical communication with the active electrode. The one or more return electrodes may be disposed on an inner surface of colpotomy cup. The one or more return electrodes may be disposed on an outer surface of colpotomy cup. The distal tip may include an electrically conductive surface that is disposed in electrical communication with the active electrode. The electrically conductive surface of the distal tip may be configured to act as a return electrode.

In various aspects, the active electrode may be configured to axially translate relative to the colpotomy cup. The uterine manipulator may support a drive mechanism that is operably coupled to the active electrode. The drive mechanism may be actuatable to enable the active electrode to distally translate to a position that extends beyond a distal end face of the colpotomy cup.

In some aspects, the distal tip may support an inflatable balloon.

In various aspects, the colpotomy cup may include a platform assembly including elongated rods that longitudinally space the active electrode from a distal end face of the colpotomy cup.

According to another aspect, a robotic colpotomy system includes a robotic arm, an instrument drive unit supported on the robotic arm, and a uterine manipulator coupled to the instrument drive unit and defining a longitudinal axis. The uterine manipulator has a proximal housing assembly and an elongated shaft assembly that extends distally from the proximal housing assembly. The elongated shaft assembly supports a colpotomy assembly on a distal end portion thereof. The colpotomy assembly includes a colpotomy cup and extends distally to a distal tip. The colpotomy cup is rotatable relative to the proximal housing assembly in response to actuation of the instrument drive unit.

In aspects, the colpotomy cup may include one or more protrusions on a distal end face thereof. The one or more protrusions may be positioned to delineate a vaginal fornix as the colpotomy cup rotates about the longitudinal axis of the uterine manipulator.

In some aspects, the colpotomy cup may include lights positioned to rotate with the colpotomy cup. The lights may be positioned to delineate a vaginal fornix as the colpotomy cup rotates about the longitudinal axis of the uterine manipulator.

In aspects, the colpotomy cup may include one or more sidewalls and a tubular cutting member supported adjacent to the one or more sidewalls and rotatable relative to the one or more sidewalls.

In various aspects, the colpotomy cup may include a plurality of leaflets. The plurality of leaflets may be removably coupled to the distal tip. The plurality of leaflets may be coupled to a gear assembly that is rotatable relative to the distal tip to move the leaflets relative to one another.

According to still another aspect, a robotic colpotomy system includes a robotic arm, an instrument drive unit supported on the robotic arm, and a uterine manipulator supported on the robotic arm and defining a longitudinal axis. The uterine manipulator is coupled to the instrument drive unit and has an elongated shaft assembly. The elongated shaft assembly supports a colpotomy assembly on a distal end portion thereof. The colpotomy assembly includes a colpotomy cup and extends distally to a distal tip. The colpotomy cup is axially movable relative to the distal tip in response to actuation of the instrument drive unit.

According to yet another aspect, a robotic colpotomy system includes a robotic arm, an instrument drive unit supported on the robotic arm, and a uterine manipulator supported on the robotic arm and defining a longitudinal axis. The uterine manipulator is coupled to the instrument drive unit and has an elongated shaft assembly. The elongated shaft assembly supports a colpotomy assembly on a distal end portion thereof. The colpotomy assembly includes a colpotomy cup and extends distally to a distal tip. The distal tip is movable relative to colpotomy cup in response to actuation of the instrument drive unit.

In aspects, the colpotomy cup may include a plurality of leaflets, wherein the distal tip is axially movable relative to the colpotomy cup to move the plurality of leaflets from a first position to a second position. In aspects, the distal tip may rotate relative to the colpotomy cup to move the plurality of leaflets from a first position to a second position.

According to a further aspect, a colpotomy cup system includes a plurality of leaflets configured to define a diameter of the colpotomy cup and a cinch coupled to the plurality of leaflets. The cinch is actuatable to move the plurality of leaflets from a first position to a second position to change the diameter of the colpotomy cup.

In aspects, in the first position, adjacent leaflets of the plurality of leaflets may be spaced apart from one another, and in the second position, the adjacent leaflets may be positioned to contact one another.

According to still another aspect, a uterine manipulator includes an elongated shaft assembly and a colpotomy cup assembly supported on a distal end portion of the elongated shaft assembly. The colpotomy cup assembly includes a proximal colpotomy cup and a distal colpotomy cup nested within the proximal colpotomy cup. The proximal colpotomy cup extends radially outward to a first rim. The distal colpotomy cup extends radially outward to a second rim. The first and second rims are longitudinally spaced apart from one another and positioned to define an indentation therebetween. The indentation is configured to define an annular track positioned to guide an independent incising instrument therearound to effectuate a colpotomy incision.

Other aspects, features, and advantages will be apparent from the description, the drawings, and the claims that follow.

### BRIEF DESCRIPTION OF THE DRAWINGS

The accompanying drawings, which are incorporated in and constitute a part of this specification, illustrate aspects of this disclosure and, together with a general description of this disclosure given above, and the detailed description given below, serve to explain the principles of this disclosure, wherein:
FIG. 1 is a perspective view of a robotic colpotomy system in accordance with the principles of this disclosure;
FIG. 2 is an enlarged, perspective view of a proximal portion of a robotic uterine manipulator of the robotic colpotomy system of FIG. 1;
FIG. 3A is an enlarged view, in partial longitudinal cross-section, of a distal portion of the robotic uterine manipulator of FIG. 2 inserted transvaginally into a patient's uterus with a colpotomy cup of the robotic uterine manipulator disposed adjacent to a vaginal fornix of the patient and disposed in a first position;
FIG. 3B is another view of the distal portion of the robotic uterine manipulator of FIG. 2 with the colpotomy cup thereof disposed in second position;
FIG. 4 is an enlarged, perspective view of another colpotomy cup;
FIG. 5 is an enlarged, perspective view of another colpotomy cup of the robotic colpotomy system of FIG. 1;
FIGS. 6A and 6B are progressive side views of a distal portion of another robotic uterine manipulator of the robotic colpotomy system of FIG. 1;
FIG. 7 is a perspective view of still another colpotomy cup of still another robotic uterine manipulator of the robotic colpotomy system of FIG. 1;
FIGS. 8A and 8B are progressive views of a distal portion of the robotic uterine manipulator with the colpotomy cup of FIG. 7, the views illustrating the colpotomy cup rotating about a vaginal fornix of a patient;
FIG. 9 is a perspective view of yet another colpotomy cup of the robotic colpotomy system of FIG. 1;
FIG. 10 is a perspective view of still another colpotomy cup of the robotic colpotomy system of FIG. 1;
FIGS. 11A and 11B are progressive views illustrating a distal portion of yet another robotic uterine manipulator of the robotic colpotomy system of FIG. 1;
FIGS. 12A-12C are progressive views illustrating another colpotomy cup of another robotic uterine manipulator being secured to cervical tissue of a patient;
FIGS. 13A and 13B are progressive views illustrating a distal portion of still another robotic uterine manipulator of the robotic colpotomy system of FIG. 1 with a colpotomy cup thereof being moved between a first position and a second position;
FIG. 14A is a perspective view of yet another colpotomy cup of another robotic uterine manipulator of the robotic colpotomy system of FIG. 1; and
FIG. 14B is a perspective view illustrating the colpotomy cup of FIG. 14A transvaginally inserted into a patient and positioned relative to another surgical instrument.

### DETAILED DESCRIPTION

Aspects of this disclosure are described in detail with reference to the drawings, in which like reference numerals designate identical or corresponding elements in each of the several views. As used herein, the term "distal" refers to that portion of structure farther from the user, while the term "proximal" refers to that portion of structure, closer to the user. As used herein, the term "clinician" refers to a doctor, nurse, or other care provider and may include support personnel.

In the following description, well-known functions or constructions are not described in detail to avoid obscuring the present disclosure in unnecessary detail.

Robotic surgical systems have been used in minimally invasive medical procedures and can include robotic arm assemblies. Such procedures may be referred to as what is commonly referred to as "Telesurgery." Some robotic arm assemblies include one or more robot arms to which surgical instruments can be coupled. Such surgical instruments include, for example, electrosurgical forceps, cutting instruments, staplers, graspers, electrocautery devices, or any other endoscopic or open surgical devices. Prior to or during use of the robotic surgical system, various surgical instruments can be selected and connected to the robot arms for selectively actuating end effectors of the connected surgical instruments. Some of these surgical instruments utilize electrical energy, for example, to effectuate electrocautery.

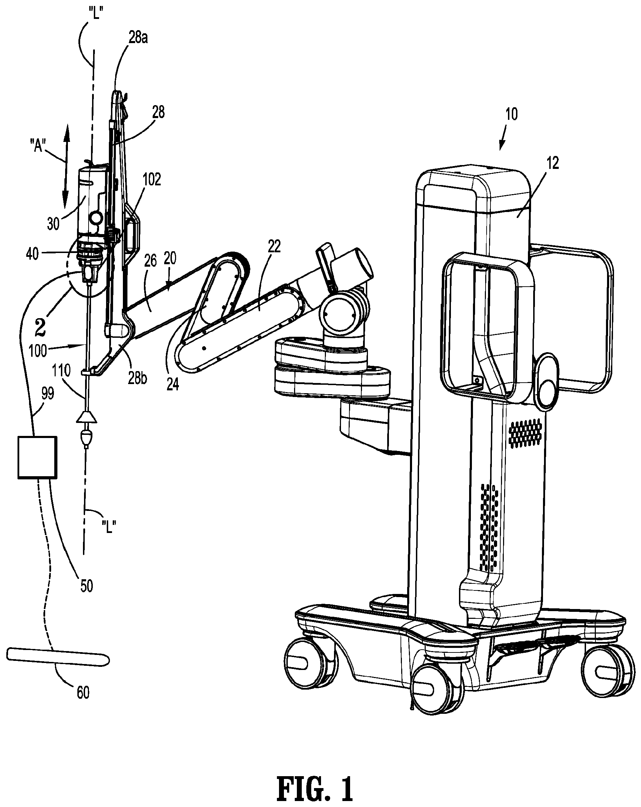

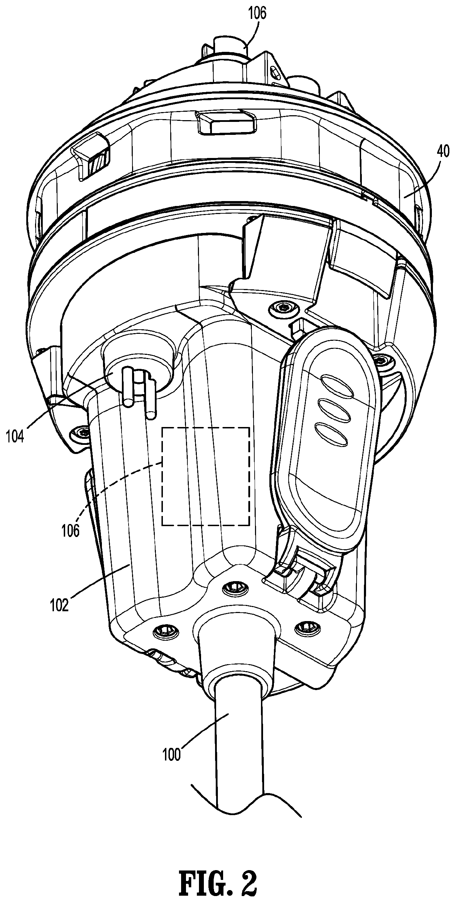

With reference to FIGS. 1 and 2, a robotic surgical system, such as the robotic colpotomy system 10 illustrated in FIG. 1, includes a robotic arm assembly 20 that supports a surgical instrument, such as a uterine manipulator 100, for effectuating a surgical procedure (e.g., a colpotomy), an instrument drive unit 30 that imparts driving force to uterine manipulator 100, and a sterile interface module 40 that enables a proximal housing assembly 102 of uterine manipulator 100 to interface with instrument drive unit 30. This interface advantageously maintains sterility, provides a means to transmit electrical communication between robotic colpotomy system 10 and uterine manipulator 100, provides a means for transferring torque (e.g., rotational force) from robotic colpotomy system 10 (e.g., IDU 30) to uterine manipulator 100 for performing a function (e.g., sealing, cutting, manipulating, etc.) with uterine manipulator 100 and/or provides a means to selectively attach/remove uterine manipulator 100 to robotic colpotomy system 10 (e.g., for rapid instrument exchange). For a more detailed description of similar sterile interface modules and components thereof, reference can be made to WO2017205308 by Zemlock et al.

Robotic colpotomy system 10 further includes an energy source such as an electrosurgical generator 50 that couples to uterine manipulator 100 and/or any number of other surgical instruments such as an electrosurgical probe or an electrocautery blade 60 via an electrosurgical cable 99 and a connector assembly 104 supported by sterile interface module 40 and/or proximal housing assembly 102 of uterine manipulator 100. For a more detailed description of one example of an electrosurgical generator, reference can be made to U.S. Patent No. 8,784,410. For a more detailed description of one example of connector assembly 104, reference can be made to U.S. Patent Application No. 62/823,036, filed March 25, 2019, and entitled "Robotic Surgical Systems with Electrical Switch of Instrument Attachment," . For a more detailed description of one example of an electrocautery blade 60, reference can be made to U.S. Patent No. 8,128,622 or U.S. Patent No. 8,460,289.

Robotic colpotomy system 10 employs various robotic elements to assist the clinician and allow remote operation (or partial remote operation) of surgical instrumentation such as uterine manipulator 100. Various robotic arms, gears, cams, pulleys, electric and mechanical motors, etc. may be employed for this purpose and may be designed with robotic colpotomy system 10 to assist the clinician during the course of an operation or treatment, and which can be included with, and/or part of one or more drive mechanisms 106 of uterine manipulator 100, sterile interface module 40, and/or instrument drive unit 30. Such robotic systems may include remotely steerable systems, automatically flexible surgical systems, remotely flexible surgical systems, remotely articulating surgical systems, wireless surgical systems, modular or selectively configurable remotely operated surgical systems, etc.

Robotic colpotomy system 10 includes a medical work station (not shown) that may be employed with one or more consoles positioned next to the operating theater or located in a remote location. In this instance, one team of clinicians may prep the patient for surgery and configure robotic colpotomy system 10 with uterine manipulator 100 while another clinician (or group of clinicians) remotely controls uterine manipulator 100 via the one or more consoles. As can be appreciated, a highly skilled clinician may perform multiple operations in multiple locations without leaving his/her remote console. This can be economically advantageous and a benefit to the patient or a series of patients. For a detailed description of exemplary medical work stations and/or components thereof, reference may be made to U.S. Patent No. 8,828,023 and PCT Application Publication No. WO2016/025132.

With continued reference to FIG. 1, robotic arm assembly 20 of robotic surgical system 10 includes a cart 12 having robotic arms 22, 24, 26 that are pivotally coupled together and movable together and/or relative to one another and cart 12. Robotic arm 26 is coupled to a slide rail 28 that supports instrument drive unit ("IDU") 30 and sterile interface module 40 for operating uterine manipulator 100. IDU 30 defines a longitudinal axis "L" and is slidably supported on slide rail 28 and selectively axially movable along longitudinal axis "L," as indicated by arrows "A," between a proximal position adjacent a proximal end portion 28a of slide rail 28, and a distal position adjacent a distal end portion 28b of slide rail 28.

Robotic surgical system 10 can be in the form of an electrosurgical colpotomy system. In general, components of the electrosurgical colpotomy system can be used to effectuate a colpotomy. Briefly, when using a uterine manipulator for colpotomy during a laparoscopic hysterectomy, a colpotomy cup can be used as a backstop for a clinician to circumferentially cut along with a laparoscopic tool (e.g., radiofrequency or "RF" tool) around the uterus. To make such a circumferential cut uniform, the clinician is required to determine the location of a rim of the colpotomy cup. Indeed, to identify the exact location of the rim, the clinician may be required to repeatedly move the uterine manipulator as the cut is made. This movement can be cumbersome and/or time consuming, particularly when clinician must also coagulate and transect uterine arteries in order to minimize blood loss during the colpotomy.

In order to improve such colpotomy procedures, uterine manipulator 100, for instance, can be used to selectively treat (e.g., coagulate, cut, etc.) tissue "T" such as vaginal or uterine tissue quickly and effectively. Briefly, uterine manipulator 100 may utilize bipolar energy to complete the colpotomy procedure safely with adequate coagulation and controlled thermal spread.

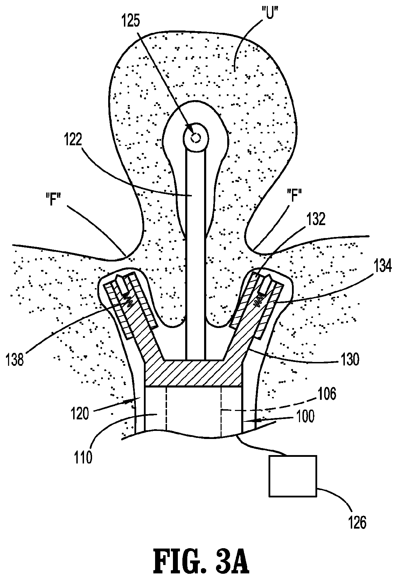

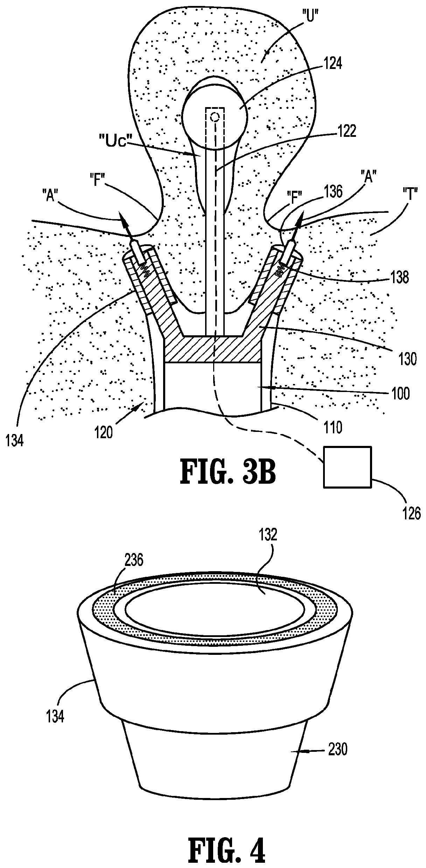

Referring to FIGS. 3A and 3B, uterine manipulator 100 includes an elongated shaft assembly 110 that extends from proximal housing assembly 102 to a distal colpotomy assembly 120. Distal colpotomy assembly 120 includes a distal tip 122 that supports an inflatable balloon 124 on a distal end portion thereof. Distal tip 122 can be conductive and may include metallic material. Distal tip 122 can also be wired to act as a return path for electrosurgical energy from energy source 50 (see FIG. 1). Distal tip 122 is configured to be inserted into a patient's uterus "UC" for inflating balloon 124 therein to secure distal colpotomy assembly 120 to a uterus "U" of the patient. Inflation balloon 124 is disposed in fluid communication with a fluid source 126 (e.g., saline) via a lumen 125 defined through distal tip 122 for inflating balloon 124. Distal colpotomy assembly 120 further includes a colpotomy cup 130 that is disposed in mechanical and/or electrical cooperation with drive mechanism 106 of uterine manipulator 100 to enable colpotomy cup 130 to treat the tissue "T" (e.g., cut and coagulate tissue for effectuating a colpotomy).

Colpotomy cup 130 of uterine manipulator 100 includes an inner return electrode 132, which may have an annular or ring configuration, supported on an inner surface thereof and an outer return electrode 134, which may have an annular or ring configuration, supported on an outer surface thereof. Inner and/or outer return electrodes 132, 134 are conductive and are configured to act as return paths for electrical energy. Although shown supported on a distal end portion of colpotomy cup 130, inner and/or outer return electrodes 132, 134 can be disposed along the entirety and/or portions (continuous and/or discontinuous) of colpotomy cup 130. Inner and/or outer return electrode 132, 134 may include metallic material and/or may be incorporated in and/or layered/coated on inner and/or outer surfaces of colpotomy cup 130. Inner and/or outer return electrodes 132, 134 may be wired to energy source 50. Colpotomy cup 130 further includes an active electrode 136 that may be axially (e.g., distally) advanced relative to colpotomy cup 130, as indicated by arrows "A" (FIG. 3B) to enable active electrode 136a to cut through and coagulate the vaginal fornix "F" of the patient as colpotomy cup 130 and/or active electrode 136 is/are advanced through fornix "F" to create the colpotomy when active electrode 136 is electrically activated. Colpotomy cup 130 may include an actuating mechanism 138 that may be operatively coupled to drive mechanism 106 to enable extension and/or retraction of active electrode 136 relative to colpotomy cup 130 to effectuate a 360 degree cut (e.g., all 360 degree simultaneously) in tissue via axial advancement of active electrode 136a. Additionally and/or alternatively, active electrode 136 can be translated and rotated and/or just rotated to effectuate the 360 degree cut. In aspects, colpotomy cup 130 and/or components thereof, can be rotated and/or translated relative to (and/or with) elongated shaft assembly 110 and/or proximal housing assembly 102 of uterine manipulator 100 to effectuate functions of colpotomy cup for treating tissue (e.g., cutting and/or coagulating). Actuating mechanism 138 can include, for example, any suitable electromechanical components (e.g., a spring, a gear, lever, switch, etc.) to effectuate movement of active electrode 136 relative to colpotomy cup 130.

In some aspects, as seen in FIG. 4, a colpotomy cup 230 of uterine manipulator 100 includes an active electrode 236 fixed to a distal end thereof and disposed between return electrodes 132, 134. Active electrode 236, like return electrodes 132, 134, is disposed in electrical communication with energy source 50 so that colpotomy cup 230 can be used just to coagulate blood vessels and/or uterine arteries adjacent fornix "F." In particular, with active electrode 236 adjacent to (e.g., concentric with) return electrodes 132, 134, electrical energy (e.g., RF energy) can travel between the active and return electrodes 236, 132, 134 to coagulate the tissue "T" before creating a cut with monopolar energy from a laparoscopic RF tool such as electrocautery blade 60.

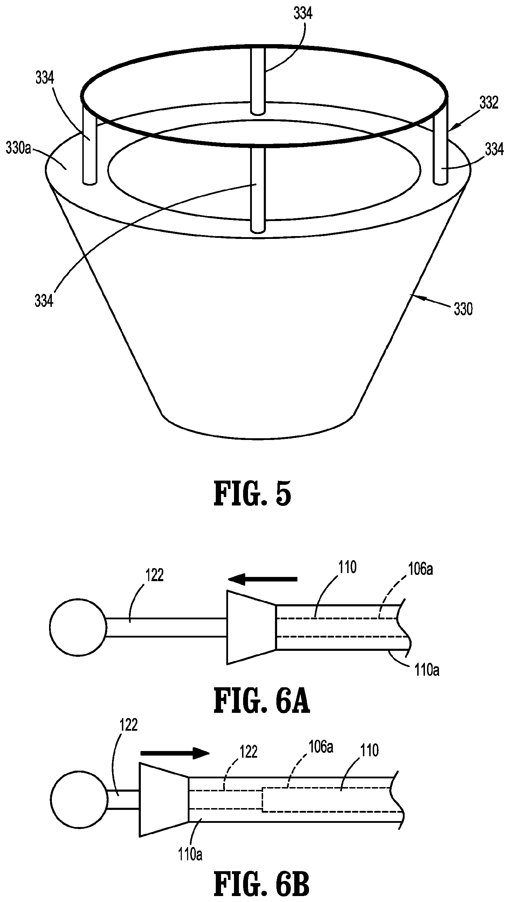

With reference to FIG. 5, in some aspects, a colpotomy cup 330 includes a platform assembly 332 extending from a distal end face 330a of colpotomy cup 330. Platform assembly 332 includes elongated rods 334 that are circumferentially supported in spaced-apart relation about the distal end of colpotomy cup 330 and coupled together by an active electrode 336. Active electrode 336 may be an electrode wire in the form of an annular ring. Active electrode 336 is spaced from distal end face 330a of colpotomy cup 330 and positioned to prevent buildup of eschar when cutting tissue "T" (e.g., a colpotomy incision) and control energy flow (and thermal spread) for artery coagulation. Platform assembly 332 is electrically coupled to energy source 50 to enable active electrode 336 to conduct electrical or RF energy (e.g., a hot wire) to effectuate a colpotomy.

By attaching uterine manipulator 100 to robotic arm assembly 20 of robotic surgical system 10, for example, additional advantages and capabilities that are not necessarily cost effective or viable with a manually operated uterine manipulator are achieved. The following aspects of this disclosure particularly benefit from such motion/force.

Turning now to FIGS. 6A and 6B, in aspects, elongated shaft assembly 110, or portions thereof (e.g., an outer tube 110a), can be axially movable relative to distal tip 122 and/or relative to an inner or central shaft 106a of drive mechanism 106.

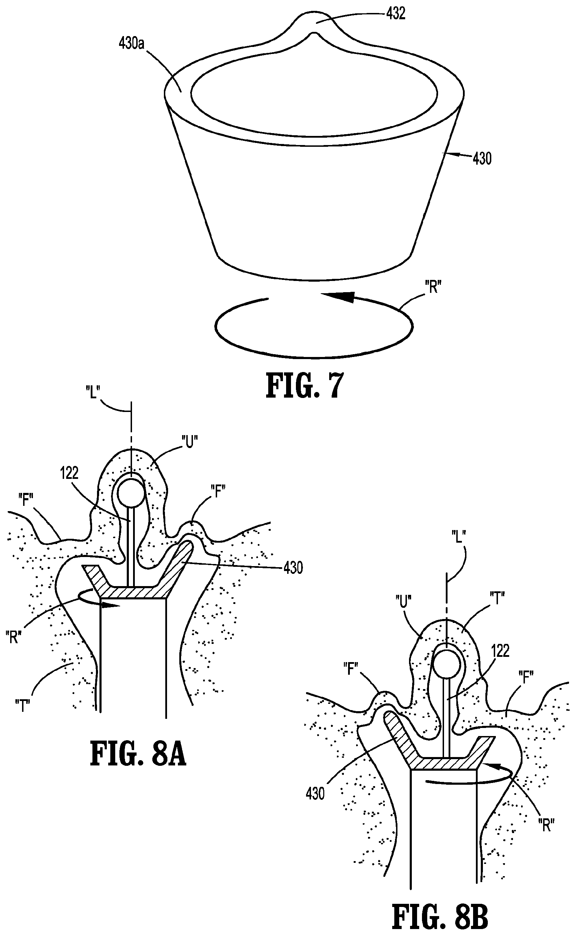

With reference to FIGS. 7, 8A, and 8B, colpotomy cup 430 can be rotated about longitudinal axis "L" and about distal tip 122, as indicated by arrows "R." Colpotomy cup 430 includes one or more protrusions 432 that extend from a distal end face 430a of colpotomy cup 430 that are positioned to help identify a location and/or orientation of colpotomy cup 430 in vivo. Each protrusion 432 can have any suitable geometry. Protrusion 432 can be positioned to raise or delineate the fornix "F," or portions thereof, as protrusion 432 rotates about the patient's uterus "U" (e.g., mouse under the rug effect).

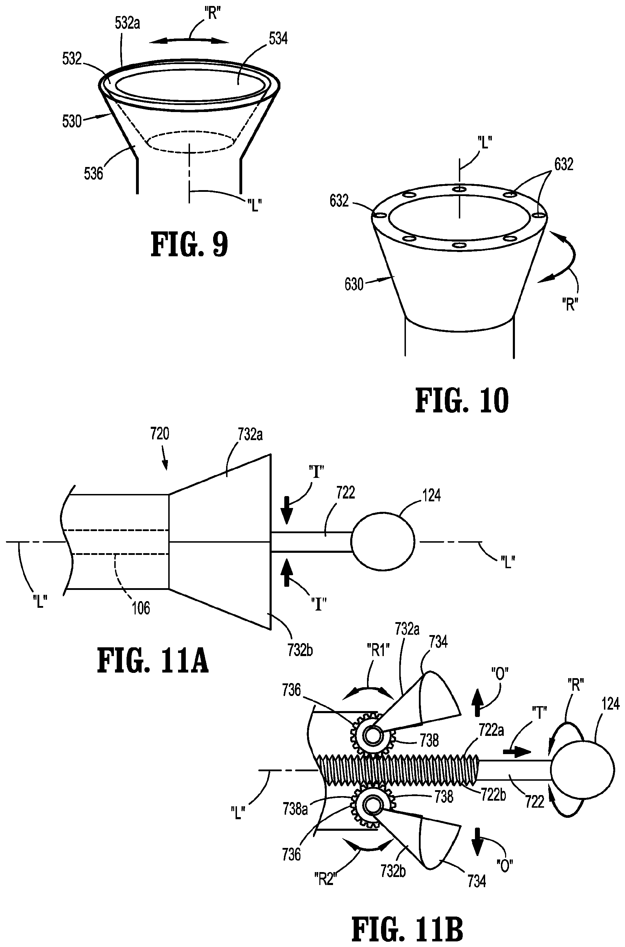

Turning now to FIG. 9, colpotomy cup 530 can include a tubular cutting member 532, such an RF wire, probe, circular knife, with a cutting edge 532a rotatably supported between an inner wall 534 and an outer wall 536 of colpotomy cup 530. In particular, tubular cutting member 532 is rotatable about longitudinal axis "L", as indicated by arrows "R," to help create a colpotomy incision as tubular cutting member 532 rotates relative to inner and outer walls 534, 536 of colpotomy cup 530.

As seen in FIG. 10, colpotomy cup 630 can include lights 632, such as LEDs, supported thereon. Colpotomy cup 630 can be rotated to rotate lights 632 about longitudinal axis "L", and as indicated by arrows "R" to facilitate delineation of, for example, the vaginal fornix "F," while uterine manipulator 100 is positioned in vivo.

Turning now to FIGS. 11A and 11B, a distal colpotomy assembly 720 includes a rotatable distal tip 722 supporting inflation balloon 124 and coupled to drive assembly 106. Rotatable distal tip 722 is rotatable about longitudinal axis "L," as indicated by arrows "R." In some aspects, distal tip 722 can be axially fixed. In other aspects, distal tip 722 can axially translate, as indicated by arrows "T" as distal tip 722 rotates. Distal tip 722 includes a threaded portion 722a having threads 722b, which may be in the form of a worm gear. Distal colpotomy assembly 720 includes a colpotomy cup 730 having leaflet assemblies 732a, 732b that are separate and independent of one another. Although only two leaflet assemblies 732a, 732b are shown, any number of leaflet assemblies can be provided (3, 4, 5 . . . n). Each leaflet assembly 732a, 732b includes a leaflet 734 and a gear assembly 736 coupled to leaflet 734. Gear assembly 736 is engaged with threaded portion 722a of distal tip 722 to enable gear assembly 736 to rotate in response to rotation of rotatable distal tip 722 about longitudinal axis "L." More specifically, gear assembly 736 include gears 738 with teeth 738a that enmesh with threads 722b of rotatable distal tip 722. Rotation of gears 738, as indicated by arrows "R1" and "R2" cause leaflets 734 of respective leaflet assemblies 732a, 732b to rotate toward (e.g., radially inward) or away (e.g., radially outward) from one another and relative to longitudinal axis "L" as indicated by arrows "I" and "O," respectively. In this regard, a size of a diameter of colpotomy cup 730 can be actively adjusted as desired.

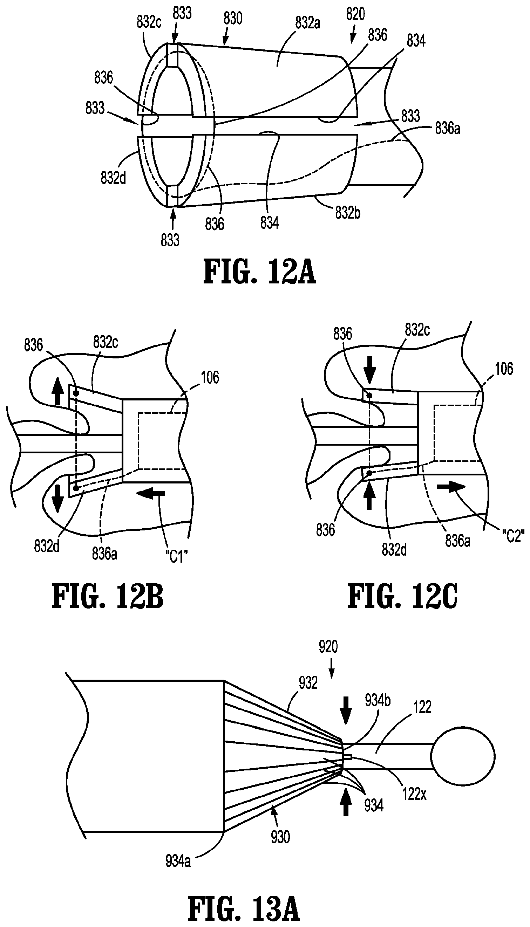

With reference to FIGS. 12A-12C, a distal colpotomy assembly 820 includes a colpotomy cup 830 having a plurality of leaflets 832a, 832b, 832c, and 832d that are radially spaced apart from one another about longitudinal axis "L" (see FIGS. 12A and 12B) by gaps 833 defined between sidewalls 834 of adjacent leaflets of the plurality of leaflets 832a, 832b, 832c, and 832d. Although only four leaflets are illustrated, colpotomy cup 830 can include any number of leaflets. Distal colpotomy assembly 820 further includes a cinch 836 thread through sidewalls 834 of the plurality of leaflets 832a, 832b, 832c, and 832d. Cinch 836 is coupled to drive assembly 106 and actuatable via movement of drive assembly 106, as indicated by arrows "C1" and "C2" to tighten and/or loosen cinch 836 (or a tether 836a thereof) to selectively and simultaneously move the plurality of leaflets 832a, 832b, 832c, and 832d toward (e.g., radially inward relative to longitudinal axis "L") and/or away (e.g., radially outward relative to longitudinal axis "L") from one another to change a diameter of colpotomy cup 830 for selectively clamping on the cervix of a patient, for example.

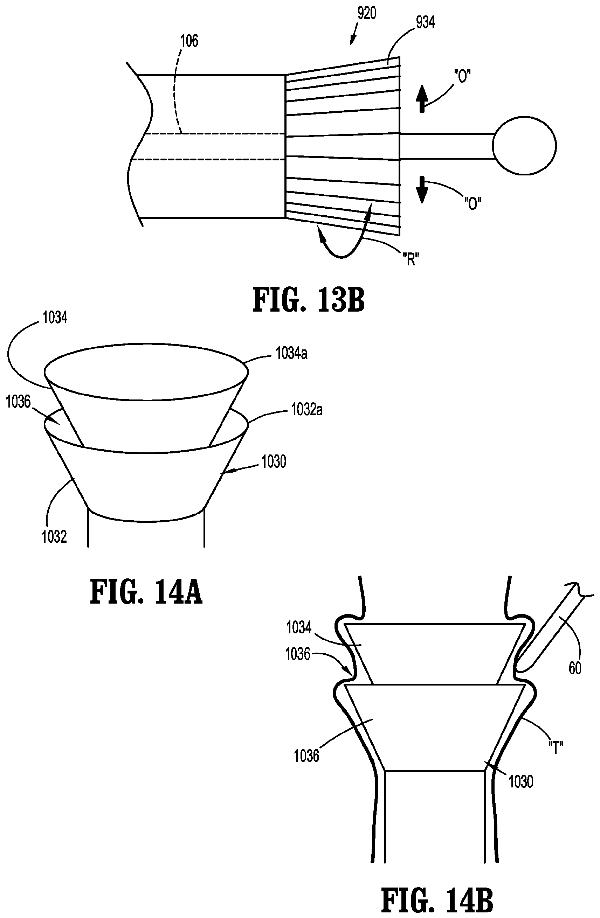

Turning now to FIGS. 13A and 13B, a distal colpotomy assembly 920 includes a colpotomy cup 930 having a series of leaflets 934 that are fixedly coupled together. Each leaflet 934 has a proximal end portion 934a coupled to elongated shaft assembly 110 and a distal end portion 934b removably coupled to distal tip 122 to reduce a profile and/or distal diameter of colpotomy cup 930, for example, to facilitate transvaginal insertion of uterine manipulator 100. Distal end portion 934b can be coupled to distal tip 122 via any suitable mechanical, electrical, and/or chemical fastening technique such as magnetics, adhesive, friction-fit, etc. For example, distal end portion 934b can be secured to distal tip 122 by a frangible feature 122x that is configured to break upon a rotation of colpotomy cup 930 relative to distal tip 122, as indicated by arrows "R," so that distal end portions 934b of leaflets 934 can flare radially outward, as indicated by arrows "O" in response to centrifugal forces to open colpotomy cup 930. As noted above frangible feature 122x can be magnetic connection and/or an adhesive, the binding forces of which are configured to break or other enable disconnection of distal end portion 934b of leaflets 934 from distal tip 122 upon rotational movement of colpotomy cup 930. As can be appreciated, rotational movement of colpotomy cup 930 can be effectuated by actuation of drive assembly 106, which can be coupled directly or indirectly with colpotomy cup 930. In some aspects, distal tip 122 may be axially advanceable relative to colpotomy cup 930 to enable colpotomy cup 930 to separate or flex away from distal tip 122. In aspects, colpotomy cup 930 can be formed of any suitable material to enable colpotomy cup 930 to bias from a radially contracted position (FIG. 13A) to a radially expanded position (FIG. 13B). In aspects, colpotomy cup 930 may include shape memory material such as nickel titanium alloy.

With reference also to FIGS. 14A and 14B, a colpotomy cup 1030 can include a proximal colpotomy cup 1032 and a distal colpotomy cup 1034, each with a funnel configuration. Proximal colpotomy cup 1032 supports distal colpotomy cup 1034 in a nested arrangement so that respective outer rims 1032a, 1034a thereof are disposed in a longitudinal spaced apart arrangement to define an indentation 1036 longitudinally between outer rims 1032a, 1034a. Indentation 1036 functions to enable probe 60 of robotic surgical system 10 to have a path or track along which electrosurgical probe 60 can easily follow to make a colpotomy incision in the patient's tissue "T."

Securement of any of the components of the disclosed devices may be effectuated using known securement techniques such welding, crimping, gluing, fastening, etc.

Persons skilled in the art will understand that the structures and methods specifically described herein and shown in the accompanying figures are non-limiting exemplary aspects, and that the description, disclosure, and figures should be construed merely as exemplary of particular aspects. It is to be understood, therefore, that this disclosure is not limited to the precise aspects described, and that various other changes and modifications may be effectuated by one skilled in the art without departing from the scope of the disclosure. Additionally, the elements and features shown or described in connection with certain aspects may be combined with the elements and features of certain other aspects without departing from the scope of this disclosure, and that such modifications and variations are also included within the scope of this disclosure. Accordingly, the subject matter of this disclosure is not limited by what has been particularly shown and described.

## Claims

1. An electrosurgical colpotomy system (10), comprising:
an energy source (50); and
a uterine manipulator (100) extending to a distal tip (122) and supporting a colpotomy cup (130), the colpotomy cup having an active electrode (136, 236) in electrical communication with the energy source, the active electrode positioned to treat a circumference of tissue in contact therewith, **characterised in that** the colpotomy cup includes at least one return electrode (132, 134) positioned thereon in electrical communication with the active electrode.

2. The electrosurgical colpotomy system of claim 1, wherein the at least one return electrode is disposed on an inner surface of colpotomy cup.

3. The electrosurgical colpotomy system of claim 2, wherein the at least one return electrode is disposed on an outer surface of colpotomy cup.

4. The electrosurgical colpotomy system of claim 2, wherein the distal tip includes an electrically conductive surface that is disposed in electrical communication with the active electrode.

5. The electrosurgical colpotomy system of claim 2, wherein the electrically conductive surface of the distal tip is configured to act as a return electrode.

6. The electrosurgical colpotomy system of claim 1, wherein the active electrode is configured to axially translate relative to the colpotomy cup.

7. The electrosurgical colpotomy system of claim 6, wherein the uterine manipulator supports a drive mechanism (106) that is operably coupled to the active electrode, the drive mechanism actuatable to enable the active electrode to distally translate to a position that extends beyond a distal end face of the colpotomy cup.

8. The electrosurgical colpotomy system of claim 1, wherein the distal tip supports an inflatable balloon (124).

9. The electrosurgical colpotomy system of claim 1, wherein the colpotomy cup includes a platform assembly (332) including elongated rods (334) that longitudinally space the active electrode from a distal end face of the colpotomy cup.

## Patentansprüche

1. Elektrochirurgisches Kolpotomiesystem (10), umfassend:
eine Energiequelle (50); und
einen Uterusmanipulator (100), der sich zu einer distalen Spitze (122) erstreckt und einen Kolpotomiebecher (130) trägt, wobei der Kolpotomiebecher eine aktive Elektrode (136, 236) in elektrischer Verbindung mit der Energiequelle aufweist, wobei die aktive Elektrode positioniert ist, um einen Umfang von damit in Kontakt stehendem Gewebe zu behandeln, **dadurch gekennzeichnet, dass** der Kolpotomiebecher mindestens eine darauf positionierte Gegenelektrode (132, 134) in elektrischer Verbindung mit der aktiven Elektrode einschließt.

2. Elektrochirurgisches Kolpotomiesystem nach Anspruch 1, wobei die mindestens eine Gegenelektrode auf einer inneren Oberfläche des Kolpotomiebechers eingerichtet ist.

3. Elektrochirurgisches Kolpotomiesystem nach Anspruch 2, wobei die mindestens eine Gegenelektrode auf einer äußeren Oberfläche des Kolpotomiebechers eingerichtet ist.

4. Elektrochirurgisches Kolpotomiesystem nach Anspruch 2, wobei die distale Spitze eine elektrisch leitfähige Oberfläche einschließt, die in elektrischer Verbindung mit der aktiven Elektrode eingerichtet ist.

5. Elektrochirurgische Kolpotomiesystem nach Anspruch 2, wobei die elektrisch leitfähige Oberfläche der distalen Spitze konfiguriert ist, um als eine Gegenelektrode zu wirken.

6. Elektrochirurgisches Kolpotomiesystem nach Anspruch 1, wobei die aktive Elektrode konfiguriert ist, um sich axial relativ zu dem Kolpotomiebecher zu verschieben.

7. Elektrochirurgisches Kolpotomiesystem nach Anspruch 6, wobei der Uterusmanipulator einen Antriebsmechanismus (106) trägt, der an die aktive Elektrode wirkgekoppelt ist, wobei der Antriebsmechanismus betätigbar ist, um der aktiven Elektrode zu ermöglichen, sich distal zu einer Position zu verschieben, die sich über eine distale Endfläche des Kolpotomiebechers hinaus erstreckt.

8. Elektrochirurgisches Kolpotomiesystem nach Anspruch 1, wobei die distale Spitze einen aufblasbaren Ballon (124) trägt.

9. Elektrochirurgisches Kolpotomiesystem nach Anspruch 1, wobei der Kolpotomiebecher eine Plattform-Anordnung (332) einschließt, die längliche Stäbe (334) einschließt, die die aktive Elektrode von einer distalen Endfläche des Kolpotomiebechers der Länge nach beabstanden.

## Revendications

1. Système de colpotomie électrochirurgicale (10), comprenant :
une source d'énergie (50) ; et
un manipulateur utérin (100) s'étendant jusqu'à une pointe distale (122) et supportant une coupelle de colpotomie (130), la coupelle de colpotomie ayant une électrode active (136, 236) en communication électrique avec la source d'énergie, l'électrode active étant positionnée pour traiter une circonférence de tissu en contact avec celle-ci, **caractérisé en ce que** la coupelle de colpotomie comporte au moins une électrode de retour (132, 134) positionnée sur celle-ci en communication électrique avec l'électrode active.

2. Système de colpotomie électrochirurgicale selon la revendication 1, dans lequel l'au moins une électrode de retour est disposée sur une surface interne de coupelle de colpotomie.

3. Système de colpotomie électrochirurgicale selon la revendication 2, dans lequel l'au moins une électrode de retour est disposée sur une surface externe de coupelle de colpotomie.

4. Système de colpotomie électrochirurgicale selon la revendication 2, dans lequel la pointe distale comporte une surface électroconductrice qui est disposée en communication électrique avec l'électrode active.

5. Système de colpotomie électrochirurgicale selon la revendication 2, dans lequel la surface électriquement conductrice de la pointe distale est configurée pour agir comme une électrode de retour.

6. Système de colpotomie électrochirurgicale selon la revendication 1, dans lequel l'électrode active est configurée pour se déplacer axialement par translation par rapport à la coupelle de colpotomie.

7. Système de colpotomie électrochirurgicale selon la revendication 6, dans lequel le manipulateur utérin supporte un mécanisme d'entraînement (106) qui est accouplé de manière fonctionnelle à l'électrode active, le mécanisme d'entraînement étant actionnable pour permettre à l'électrode active de se translater distalement jusqu'à une position qui s'étend au-delà d'une face d'extrémité distale de la colpotomie coupe.

8. Système de colpotomie électrochirurgicale selon la revendication 1, dans lequel la pointe distale supporte un ballon gonflable (124).

9. Système de colpotomie électrochirurgicale selon la revendication 1, dans lequel la coupelle de colpotomie comporte un ensemble plate-forme (332) comportant des tiges allongées (334) qui espacent longitudinalement l'électrode active d'une face d'extrémité distale de la coupelle de colpotomie.
